# EUROPÄISCHE PATENTSCHRIFT

(11) **EP 2 799 390 B1**
(45) Veröffentlichungstag und Bekanntmachung des Hinweises auf die Patenterteilung: **07.06.2017**
(21) Anmeldenummer: 14164436.9
(22) Anmeldetag: 11.04.2014
(51) Int. Cl.: B67B 1/00, A61M 39/02

(54) **Verfahren zum Einfügen zumindest eines Septums**
Method for inserting at least one septum
Procédé d'insertion sur au moins un septum

(30) Priorität: 29.04.2013 DE 102013104360
(43) Veröffentlichungstag der Anmeldung: 05.11.2014
(73) Patentinhaber: Gerresheimer Regensburg GmbH, 93047 Regensburg (DE)
(72) Erfinder: Bösl, Richard, 92442 Wackersdorf (DE); Schönberger, Vitus, 92269 Fensterbach (DE)
(74) Vertreter: Hannke, Christian

(56) Entgegenhaltungen:
- EP-A1- 1 717 188
- EP-A1- 1 815 881
- WO-A1-2007/140029

## Beschreibung

Die vorliegende Erfindung betrifft ein Verfahren zum Einfügen zumindest eines Septums in eine Setzöffnung eines Trägerelements, gemäß dem Oberbegriff des Patentanspruchs 1. Darüber hinaus betrifft die Erfindung eine Vorrichtung zum Einfügen zumindest eines Septums gemäß dem Oberbegriff des Patentanspruchs 10. Insbesondere kann das Trägerelement mechanisch fest an einem Transporttisch befestigt sein.
Es sind aus dem Stand der Technik verschiedene Verfahren zum Einfügen zumindest eines Septums in eine Setzöffnung eines Trägerelements bekannt. Siehe Dokumente EP 1717188 A1, EP 1815881 A1 und WO2007/140029 A1. Bei derartigen Verfahren wird das Septum beispielsweise in die Setzöffnung eingelegt und anschließend mit der Setzöffnung und/oder innerhalb dieser Setzöffnung verklebt um eine mechanisch stabile Verbindung zwischen dem Septum und dem Trägerelement herstellen zu können. Insbesondere ist bei derartigen Verfahren das Septum während des Einsetzens in die Öffnung nicht unter Vorspannung.
Derartige Verfahren sind jedoch in Ihrer Handhabung verhältnismäßig aufwendig, da ein Einsetzten und ein späteres Verkleben, wie obig bereits erwähnt, jeweils zwei voneinander getrennte Verfahrensschritte umfasst und somit ein derartiges Verfahren insbesondere nicht als ein vollautomatisches Verfahren durchgeführt werden kann. Insofern sind die aus dem Stand der Technik bekannten Verfahren besonders zeitaufwändig und kostenintensiv.

Es ist daher eine Aufgabe der vorliegenden Erfindung ein Verfahren zum Einfügen eines Septums in eine Setzöffnung eines Trägerelements bereitzustellen, welches sowohl kostengünstig als auch zeitsparend, und insbesondere vollautomatisch durchführbar ist.

Diese Aufgabe wird durch den Gegenstand des Patentanspruchs 1 gelöst.

Vorteilhafte Ausführungsformen sind in den abhängigen Ansprüchen beschrieben.

Um nun ein Verfahren zum Einfügen zumindest eines Septums in eine Setzöffnung eines Trägerelements anzugeben, welches sowohl kostengünstig als auch zeitsparend ist, macht die vorliegende Erfindung unter anderem von der Idee gebrauch, das Septum in einem ersten Schritt mittels eines Saugdorns an diesem durch Ansaugen zu befestigen. Dabei wird in einem zweiten Schritt das Septum über einer Öffnung einer in vertikaler Richtung zwischen dem Saugdorn und dem Trägerelement angeordneten Setzbuchse angeordnet und nach einem bündigen Anordnen der Setzbuchse auf dem Trägerelement das Septum, beispielsweise vollständig, durch eine sich ausgehend von der Öffnung in Richtung des Trägerelements in ihren Querschnitt verjüngenden Setzkanal der Setzbuchse mittels des Saugdorns hindurch geschoben. Dabei wird nach dem Hindurchschieben des Septums durch den Setzkanal dieses in die Setzöffnung des Trägerelement eingeschoben, wobei während des Hindurchschiebens des Septums durch den Setzkanal das Septum zumindest in lateraler Richtung elastisch verdichtet wird, um das Septum in der Setzöffnung einzuspannen.

Dabei bedeutet in diesem Zusammenhang "vertikale Richtung" eine Richtung senkrecht zu einer Haupterstreckungsebene des Transporttisches. Entsprechend bedeutet "laterale Richtung" eine Richtung parallel zur Haupterstreckungsebene des Transporttisches. Mit anderen Worten wird beginnend bei der Öffnung der Setzbuchse in vertikaler Richtung das Septum immer stärker elastisch verdichtet, sodass eine maximale Vorspannung des Septums beim Eintritt des Septums in die Setzöffnung vorliegt und diese Vorspannung des Septums daher mit in die Setzöffnung übertragen wird, sodass nach dem Entfernen des Saugdorns von dem Septum dieses bündig und fest eingespannt in der Setzöffnung verbleibt.

Ein beispielsweise aufwändiges späteres Verkleben des Septums innerhalb der Setzöffnung oder ein mechanisches Bördeln kann damit eingespart werden, da das Septum bereits vollständig und mechanisch fest durch die elastische Verdichtung und Vorspannung innerhalb der Setzöffnung mit dem Trägerelement fest verbunden ist. Vorzugsweise weist im entspannten Zustand des Septums dieses in einer Draufsicht eine größere Querschnittsfläche auf als die Setzöffnung selbst, sodass einzig und allein über die oben angesprochene elastische Verdichtung das Septum in die Setzöffnung eingesetzt werden kann.

Insofern ist mittels des hier beanspruchten Verfahrens über das Zusammenspiel aus dem Ansaugen des Septums durch den Saugdorn in Verbindung mit dem sich in vertikaler Richtung, beispielsweise konisch, verjüngenden Setzkanal ein besonders einfaches und kostengünstiges Verfahren offenbart, bei dem beispielsweise innerhalb eines vollautomatischen Prozesses, ohne weitere Hilfsmittel oder zusätzliche Verfahrensschritte, das Septum in die Setzöffnung des Trägerelements besonders einfach eingesetzt werden kann und dort wiederum ohne weitere Hilfsmittel mechanisch fest in das Trägerelement und innerhalb der Öffnung eingespannt ist.

Gemäß zumindest einer Ausführungsform ist bei dem Verfahren zum Einfügen zumindest eines Septums in eine Setzöffnung eines Trägerelements das Trägerelement mechanisch fest an einem Transporttisch befestigt, wobei in einem ersten Schritt das Septum zunächst mittels eines Saugdorns an diesem durch Ansaugen befestigt wird. In einem zweiten Schritt wird das Septum über eine Öffnung einer in vertikaler Richtung zwischen dem Saugdorn und dem Trägerelement angeordneten Setzbuchse angeordnet und nach einem bündigen Anordnen der Setzbuchse auf dem Trägerelement, das Septum vollständig durch eine sich ausgehend von der Öffnung in Richtung des Trägerelements in ihrem Querschnitt verjüngenden Setzkanal der Setzbuchse mittels des Saugdorns hindurchgeschoben. Dadurch wird das Septum in die Setzöffnung des Trägerelements eingeschoben, wobei während des Hindurchschiebens des Septums durch den Setzkanal dieses zumindest in lateraler Richtung elastisch verdichtet wird, um das Septum in der Setzöffnung einzuspannen.

Gemäß zumindest einer Ausführungsform wird nach dem Einschieben des Septums in die Setzöffnung des Trägerelements zuerst die Setzbuchse von dem Trägerelement und erst anschließend der Saugdorn von dem Septum entfernt. Insofern wird besonders einfach sichergestellt, dass der Saugdorn auf das Septum drückt, während zuerst die Setzbuchse von dem Trägerelement abgehoben wird. Damit wird gewährleistet, dass das gesamte Septum von der Setzbuchse freigegeben wird und erst nach dem Freisetzen des Septums durch die Setzbuchse auch der Saugdorn von dem Septum abgehoben wird. Dies ist insbesondere insofern vorteilhaft, als das eine Septumdicke, d. h. eine Ausdehnung des Septums in vertikaler Richtung, vorzugsweise größer als eine Ausdehnung der Setzöffnung, beispielsweise eine Bohrungstiefe (für den Fall das die Setzöffnung in Form einer Bohrung ausgeführt ist), ist, sodass nach einer derartigen "Abhebesequenz" der Setzbuchse und des Saugdorns das Septum nicht nur fest in der Setzöffnung des Trägerelements eingespannt bleibt, sondern auch vermieden werden kann, dass dieses aus der Setzöffnung herausragt.

Gemäß zumindest einer Ausführungsform werden nach dem Einschieben des Septums in die Setzöffnung des Trägerelements die Setzbuchse von dem Trägerelement und der Saugdorn von dem Septum gleichzeitig entfernt. "Gleichzeitig entfernt" heißt, dass im Rahmen von Bewegungs- und Zeittoleranzen beide Elemente im selben Moment angehoben werden. Dies bietet die Möglichkeit einer Zeitersparnis im Einsetzverfahren und kann das gesamte Verfahren in seiner Ablaufsteuerung einfacher gestalten.

Gemäß zumindest einer Ausführungsform weist zum Ansaugen und Arretieren des Septums an dem Saugdorn dieser eine Ansaugkavität auf, wobei die Ansaugkavität eine relativ zum Transporttisch sich wegkrümmende Innenwand aufweist und wobei in der Innenwand zumindest ein Ansaugkanal mündet mittels dem das Septum an die Ansaugkavität angesaugt und an dieser gehalten werden kann. Insofern bildet die sich vom Transporttisch wegkrümmende Innenwand die Ansaugkavität aus. Dazu ist denkbar, dass das Septum beispielsweise mittels einer vollautomatischen Zuführung aus einer Vereinzelung mit dem Saugdorn in Kontakt gebracht wird und durch Ansaugen, das heißt durch Erzeugen eines Unterdrucks zwischen dem Ansaugdorn und dem Septum, das Septum selbst zumindest teilweise in die oder an die Ansaugkavität an oder eingesaugt und während des Erzeugens des beispielsweise Unterdrucks, das Septum in oder an der Ansaugkavität gehalten wird. Dabei bleibt das Septum an dem Saugdorn während des ganzen Durchschiebevorgangs durch die Setzbuchse entlang und innerhalb des Setzkanals hindurch an den Saugdorn angesaugt.

Mittels der hier beanspruchten Durchmesser-Abstimmung von Saugdorn und einem Durchmesser des Setzkanals in Verbindung mit der sich wegkrümmenden Innenwand, wird verhindert, dass während des Durchführens des Septums durch den Saugkanal, beispielsweise ein unterer Schnittrand des Septums sich nach oben wölbt und zwischen dem Saugdorn und der Setzbuchse während des Hindurchführens eingeklemmt wird. Mit anderen Worten wird während des Ansaugens das Septum derart in die Ansaugkavität angesaugt, dass sich eine seitliche Wandung des Septums nach innen krümmen kann und somit der Schnittrand des Septums möglichst wenig in Kontakt mit einer Innenwandung des Setzkanals kommt. Durch eine derartige zumindest teilweise Anpassung des Septums an die äußere Form der Ansaugkavität wird daher sichergestellt, wie bereits obig erwähnt, dass zum einen das Septum besonders fest und stabil an den Saugdorn befestigt und arretiert ist und zum anderen weder Verkantungen noch sonstige Verschleißerscheinungen während des Setzens, das heißt während des Durchführens des Septums durch den sich verjüngenden Setzkanal erfolgt.

Insbesondere kann es sich bei dem Septum um ein zwei-lagiges Septum handeln, bei dem Anforderungen an eine Vorspannung und/oder die Einfügung einer Vorspannung in das Septum, einfacher als bei einlagigen Septen zu erfüllen sind beziehungsweise nicht zu hoch an ein automatisches, beispielsweise vollautomatisches, Setzverfahren sind. Dazu kann das Septum mit einem Gummi und/oder einem Moosgummi gebildet sein oder mit einem sonstigen elastisch verformbaren und/oder elastisch verdichtbares Material gebildet sein. Es hat sich herausgestellt, dass ein Gummi und/oder Moosgummi besonders einfach und ohne Materialschädigungen im Septum zu hinterlassen elastisch komprimierbar ist und darüber hinaus derartige Septen besonders kostengünstig herstellbar oder erhaltbar sind.

Gemäß zumindest einer Ausführungsform ist die Öffnung in der Setzbuchse durch eine in der Setzbuche ausgebildete in Richtung des Transporttisches sich im Querschnitt verjüngende Einsatzöffnung gebildet. Bei der Einsatzöffnung kann es sich beispielsweise um einen Arretierkegel handeln, welcher es ganz besonders stark vereinfacht das Septum mit möglichst wenig Aufwand und besonders zeitsparend in den Setzkanal über die Öffnung einzufügen und einzuführen. Mit anderen Worten sorgt die Einsatzöffnung für einen problemlosen, beispielsweise vollautomatischen Ablauf des Setzverfahrens, ohne dass Arretierungsfehler und/oder sonstige Materialschädigungen weder an dem Trägerelement noch an dem Septum und/oder einer Setzbuchse entstehen würden.

Gemäß zumindest einer Ausführungsform weist das Septum eine runde oder ovale Querschnittsfläche auf, wobei eine Querschnittsfläche des Setzkanals ebenfalls rund oder oval ausgebildet ist. Insbesondere ist eine Querschnittsfläche des Septums an die Form und Größe der Querschnittsfläche des Setzkanals angepasst, wobei eine Querschnittsfläche des Setzkanals zumindest im Bereich der Öffnung der Setzbuchse größer ist als eine Querschnittsfläche des Septums selbst, sodass sich eine derartige Größenausgestaltung der Öffnung der Setzbuchse und der Querschnittsfläche des Septums, das Septum problemlos in die Öffnung der Setzbuchse eingesetzt werden kann. Entsprechendes gilt für den hier beschriebenen Saugdorn, dessen Durchmesser vorzugsweise zumindest kleiner ist als der kleinste Durchmesser des Setzkanals und/oder der Setzöffnung. Zudem sorgt eine derartige Anpassung der äußeren Beschaffenheit des Septums an die Querschnittsfläche des Setzkanals für ein problemloses und vorzugsweise bündiges Hindurchschieben und homogenes elastisches Verdichten des Septums ohne dass innere Verspannungen das Septum in seiner Materialbeschaffenheit schädigen würden.

Gemäß zumindest einer Ausführungsform ist eine Innenfläche des Setzkanals der Setzbuchse mit einem Teflon gebildet oder mit einem Teflon beschichtet. Dazu ist denkbar, dass der Setzkanal selbst mit einem Teflon gebildet ist und dabei das Septum während des Hindurchführens durch den Setzkanal der Setzbuchse an der Innenfläche entlang gleitet. Es hat sich nämlich überraschenderweise herausgestellt, dass Teflon als Material für die Innenfläche nicht nur ganz besonders kostengünstig erhältlich ist, sondern in besonders einfacher Art und Weise Reibungsverluste und/oder durch Reibung verursachte Materialschädigungen an dem Septum verhindert werden können.

Gemäß zumindest einer Ausführungsform ist die Setzbuchse mit einer inneren Setzbuchse und einem in horizontaler Richtung, die innere Setzbuchse zumindest teilweise ummantelnden äußeren Mantel gebildet, wobei die Innenfläche des Setzkanals vollständig durch die innere Setzbuchse gebildet ist. "Horizontale" Richtung ist dabei eine Richtung in der Haupterstreckungsebene des Transporttisches. Dabei ermöglicht es der äußere Mantel, dass während des Setzens die gesamte Setzbuchse und insbesondere die innere Setzbuchse mechanisch stabilisiert ist und ein besonders genaues Arretieren über der Setzöffnung des Trägerelements ermöglicht wird, sodass nicht nur besonders mechanisch stabile Einsetzvorgänge ermöglicht werden, sondern ebenso die gesamte Anlage und damit auch die Setzbuchse selbst mechanisch hohen Belastungen standhält.

Gemäß zumindest einer Ausführungsform ist der äußere Mantel mit einem Metall, insbesondere Stahl, gebildet. Es hat sich herausgestellt, dass ein mit einem Metall gebildeter äußerer Mantel den Anforderungen an mechanische Stabilität und an eine möglichst kostengünstige Ausgestaltung der Setzbuchse in ganz besonderem Maße genügt.

Gemäß zumindest einer Ausführungsform ist das Trägerelement mit zumindest einem Kunststoff gebildet. Kunststoff hat sich als besonders geeignetes Material für das Trägerelement erwiesen, da es zum einen besonders kostengünstig herstellbar ist und zeichnet sich zum anderen insbesondere im Bereich der Medizintechnik durch die elastischen Eigenschaften und ein besonders sicheres Handling aus.

Es wird darüber hinaus eine Vorrichtung zum Einfügen zumindest eines Septums in eine Setzöffnung eines Trägerelements angegeben. Beispielsweise kann mittels der Vorrichtung ein Verfahren wie es in Verbindung mit ein oder mehreren der oben genannten Ausführungsformen beschrieben ist durchgeführt werden, d. h., die für die oben genannten Ausführungsformen beschrieben ist und durchgeführt werden. D. h. die für das hier beschriebene Verfahren aufgeführten Merkmale sind auch für die hier beschriebene Vorrichtung offenbart und umgekehrt.

Gemäß zumindest einer Ausführungsform umfasst die Vorrichtung zum Einfügen zumindest eines Septums in eine Setzöffnung eines Trägerelements einen Transporttisch an dem das Trägerelement befestigbar ist, wobei das Septum mittels eines Saugdorns an diesen durch Ansaugen befestigbar ist. In das Septum ist über eine Öffnung einer in vertikaler Richtung zwischen dem Saugdorn und dem Trägerelement angeordneten Setzbuchse und nach einem Anordnen der Setzbuchse auf dem Trägerelement das Septum vollständig durch einen sich ausgehend von der Öffnung in Richtung des Trägerelements und im Querschnitt verjüngenden Setzkanal der Setzbuchse vollständig mittels des Saugdorns hindurchschiebbar, wobei in die Setzöffnung des Trägerelements somit das Septum einschiebbar ist und wobei der Setzkanal derart ausgebildet ist, dass während des Hindurchschiebens des Septums durch den Setzkanal das Septum elastisch verdichtet wird, und wobei die Setzbuchse anschließend derart von dem Trägerelement und dem Septum entfernbar sind, dass das Septum in die Setzöffnung eingespannt bleibt.

Dabei weißt die hier beschriebene Vorrichtung die gleichen Vorteile wie im Zusammenhang mit dem hier beschriebenen Verfahren auf.

Im Folgenden wird das hier beschriebene Verfahren sowie die hier beschriebene Vorrichtung anhand von Ausführungsbeispielen und den dazugehörigen Figuren näher erläutert.

Die Figuren 1A bis 1C zeigen in verschiedenen schematisch perspektivischen Ansichten eine hier beschriebene Vorrichtung zum Einfügen zumindest eines Septums in eine Setzöffnung eines Trägerelements.

Die Figuren 2A bis 2D zeigen einzelne Verfahrensschritte zum Einfügen eines Septums in eine Setzöffnung eines Trägerelements.

In den Ausführungsbeispielen und den Figuren sind gleiche oder gleichwirkende Bestandteile jeweils mit den gleichen Bezugszeichen versehen. Die dargestellten Elemente sind nicht als maßstabsgerecht anzusehen, vielmehr können einzelne Elemente zum besseren Verständnis übertrieben groß dargestellt sein.

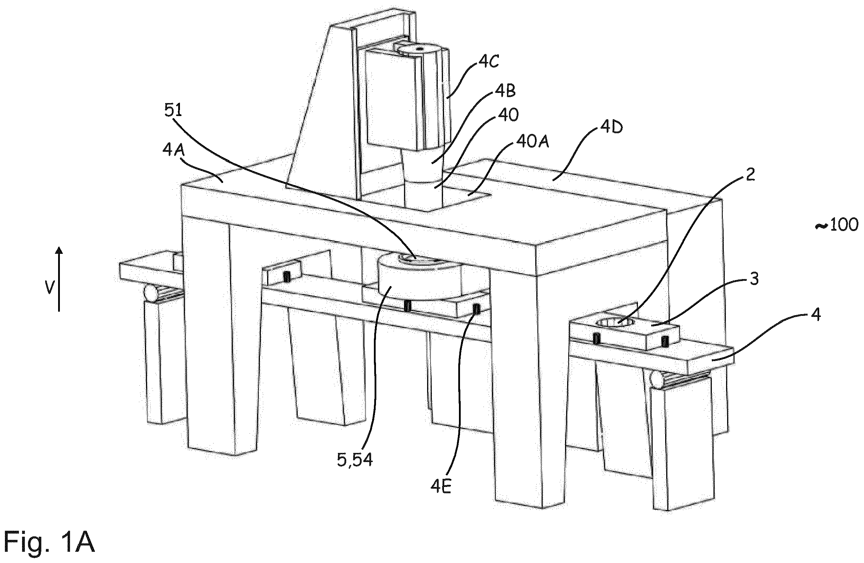

In der Figur 1A ist in einer schematisch perspektivischen Ansicht eine hier beschriebene Vorrichtung 100 zum Einfügen zumindest eines Septums 1 in eine Setzöffnung 2 eines Trägerelements 3 gezeigt. Dabei ist erkennbar, dass ein Saugdorn 40 über einen Stempel 4B und einen dazugehörigen Aktor und Aktorführung 4C fest mit einem Werkzeugtisch 4A verbunden ist, der den gesamten Saugdorn 40 mechanisch fest fixiert und stabilisiert. Dabei weist der Werkzeugtisch 4A eine Öffnung 40A auf, durch die hindurch der Saugdorn 40 und der Stempel 4B geführt und bis zu einem Transporttisch 4, auf dem das Trägerelement 3 befestigt ist, hindurchgeführt ist.

Dabei ist aus der Figur 1A und deren schematisch perspektivischen Ansicht besonders gut erkennbar, dass in vertikaler Richtung V zwischen dem Saugdorn 40 und dem Trägerelement 3 eine Setzbuchse 5 angeordnet ist, welche bündig auf dem Trägerelement 3 aufliegt. Dabei ist zudem erkennbar, das die Setzbuchse 5 einen äußeren Mantel 54 aufweist, wobei der äußere Mantel 54 und damit die gesamte Setzbuchse 5 von einer Haltevorrichtung 4D an dem gesamten Werkzeugtisch 4A fixiert ist. Dabei ist der äußere Mantel 54 vorliegend mit einem Metall, beispielsweise einem Stahl gebildet, der sich als ganz besonders widerstandsfähig und mechanisch stabil für ein insbesondere vollautomatisches Setzverfahren erwiesen hat. Insbesondere ist ein Durchmesser des Saugdorns 40 kleiner bemessen, als ein Durchmesser der Öffnung 51 der Setzbuchse 5, sodass nach dem bündigen Anordnen der Setzbuchse 5 auf dem Trägerelement 3 das Septum 1 und der Saugdorn 40 vollständig durch die Öffnung 51 in Richtung des Trägerelements 3 durch einen Setzkanal 52 hindurch mittels des Saugdorns 40 hindurchschiebbar ist und in die Setzöffnung 2 des Trägerelements 3 eingeschoben werden kann, wobei während des Hindurchschiebens des Septums 1 durch den Setzkanal 52 das Septum 1 elastisch verdichtet wird.

Dabei ist die Setzbuchse 5 und der Saugdorn 40 anschließend derart von dem Trägerelement 3 und dem Septum 1 entfernbar, dass das Septum 1 in der Setzöffnung 2 eingespannt bleibt. Insbesondere ist vorliegend die Vorrichtung 100 derart ausgestaltet, dass nach dem Einschieben des Septums 1 in die Öffnung 2 des Trägerelements 3 zuerst die Setzbuchse 5 von dem Trägerelement 3 und erst anschließend der Saugdorn 40 von dem Septum 1 entfernbar sind.

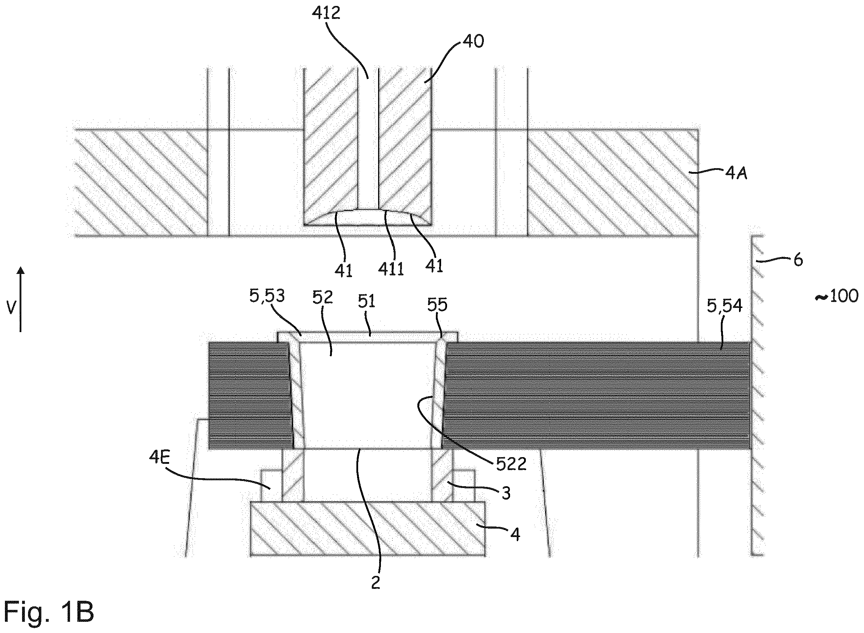

In der Figur 1 B ist in einer weiteren schematischen Seitenansicht die in der Figur 1 A gezeigte Vorrichtung 100 detailreicher dargestellt. Insbesondere ist aus der Figur 1 B besonders einfach erkennbar, dass zum Ansaugen und Arretieren des Septums 1 an dem Saugdorn 40 dieser eine Ansaugkavität 41 aufweist, wobei die Ansaugkavität 41 eine relativ zum Trägerelement 3 sich wegkrümmende Innenwand 411 aufweist und wobei in der Innenwand 411 zumindest ein Ansaugkanal 412 mündet, mittels dem das Septum 1 in die Ansaugkavität 41 eingesaugt und in dieser gehalten werden kann.

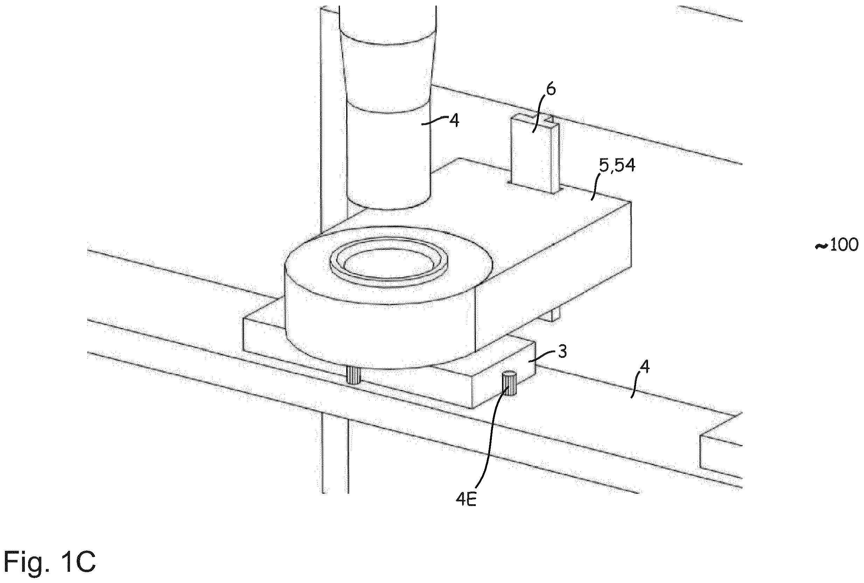

In der Figur 1C ist jeweils die in der Figuren 1A und 1B gezeigte Vorrichtung 100 nochmals in einer weiteren schematisch perspektivischen Ansicht gezeigt, bei der der Saugdorn 40 vorliegend in seinem Querschnitt rund ausgebildet ist und das gleiche ebenso sowohl für die Öffnung 51 der Setzbuchse 5 als auch für die Setzöffnung 2 gilt. Dabei ist zudem erkennbar, dass das Trägerelement 3, welches vorliegend mit einem Kunststoff gebildet ist, mittels Halterungen 4E an den Transporttisch 4 befestigt ist und somit verhindert wird, dass das Trägerelement 3, insbesondere während des Setzvorgangs, sich verschiebt, was ein Setzen des Septums 1 zumindest erschwert. Dabei ist zur besonders einfachen individuellen Höhenverstellung die Setzbuchse 5 an einer Führung 6 befestigt.

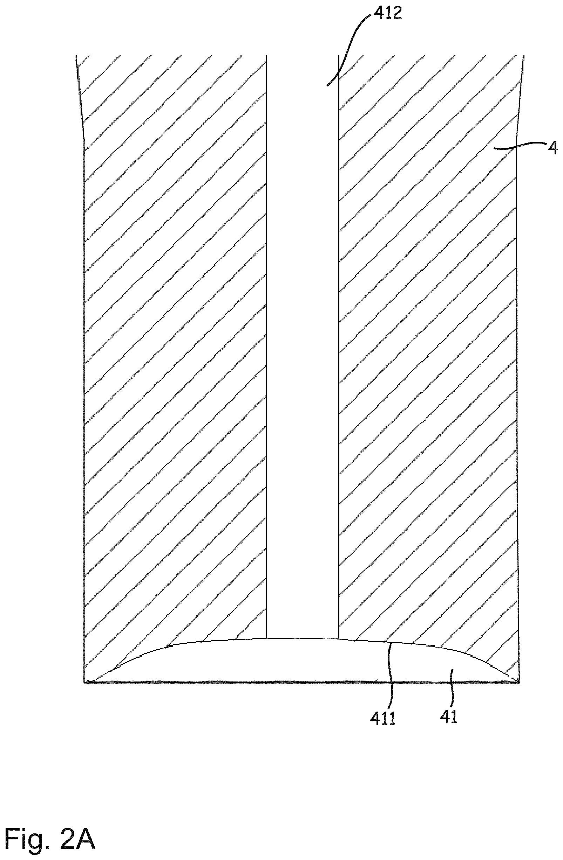

In der Figur 2A ist zunächst in einer schematischen Seitenansicht der Saugdorn 40 umfassend den bereits obig erwähnten Ansaugkanal 412 gezeigt, welcher in der Ansaugkavität 41 mündet, an welcher das Septum 1 während des Setzverfahrens angesaugt und durch den mittels des Ansaugverfahrens entstehenden Unterdruck an der Innenwand 411 der Ansaugkavität 41 und/oder an einer Berandung der Ansaugkavität 41 befestigt, gehalten und beispielsweise zumindest teilweise angeformt wird.

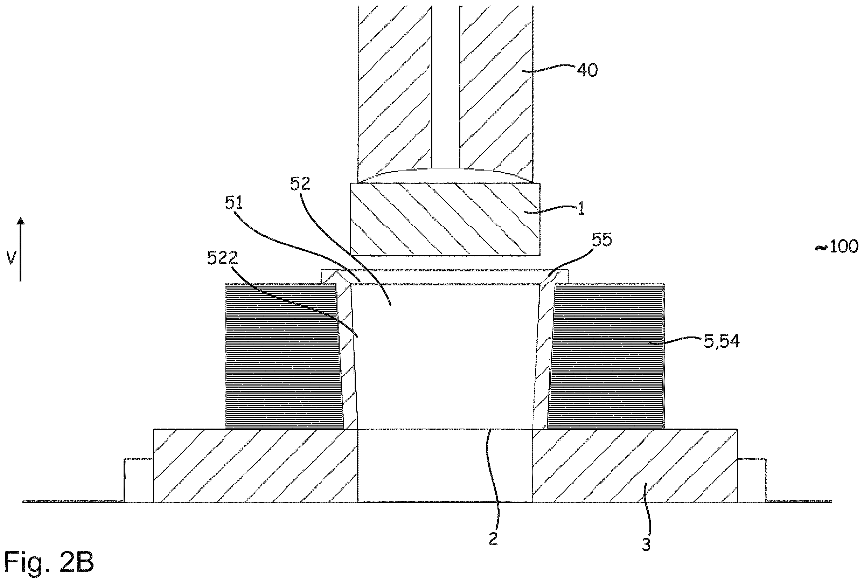

In der Figur 2B ist in einem ersten Verfahrensschritt gezeigt, wie das Septum 1 zunächst mittels des Saugdorns 40 an diesen durch Ansaugen befestigt ist und in vertikaler Richtung V überlappend mit der Öffnung 51 der Setzbuchse 5 angeordnet wird. Dabei weist die Öffnung 51 eine in der Setzbuchse 5 ausgebildete in Richtung des Transporttisches 4 sich im Querschnitt verjüngende Einsatzöffnung 55 auf. Mittels der hier beschriebenen Einsatzöffnung 55 ist es besonders einfach möglich in zeitsparender Art und Weise des Septum 1 in den Setzkanal 52 der Setzbuchse 5 einführen zu können. Wiederum ist aus der Figur 2B erkennbar, wie der äußere Mantel 54 eine innere Setzbuchse 53, welche vorliegend mit einem Teflon gebildet ist, zumindest teilweise ummantelt und mechanisch stabilisiert. Dabei kann zudem erkannt werden, dass die gesamte Setzbuchse 5 umfassend die innere Setzbuchse 53 und den äußeren Mantel 54 bündig auf den Trägerelement 3 aufliegt, wobei sich der Setzkanal 5 ausgehend von dem Saugdorn 40 in seinem Querschnitt in Richtung des Transporttisches 4 und insbesondere in Richtung des Trägerelements 3 verjüngt, wobei eine der Öffnung 51 der Setzbuchse 5 gegenüberliegende angeordnete Öffnung der Setzbuchse 5 höchstens eine Querschnitt aufweist, der der Querschnittsfläche der Setzöffnung 2 entspricht. Somit wird ein Verkanten beim Einfügen des Septum 1 in die Setzöffnung 2 vermieden.

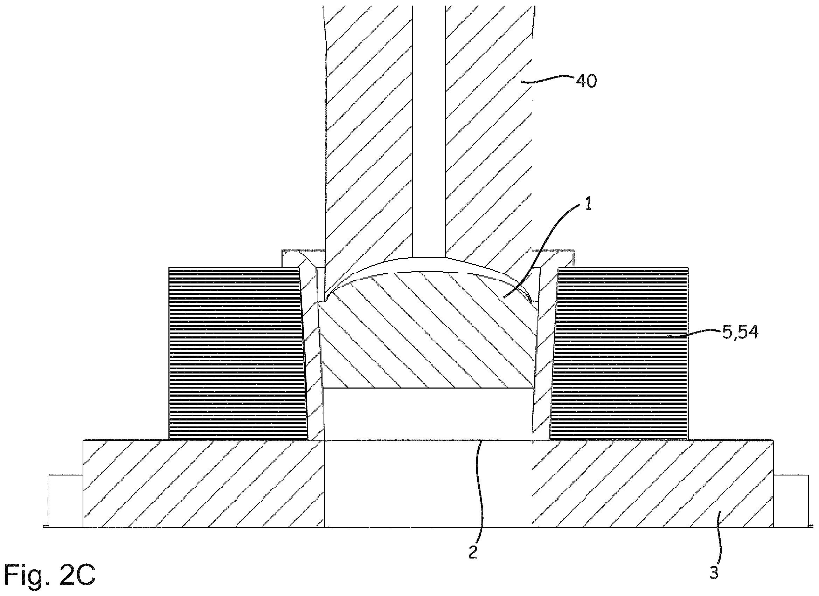

In der Figur 2C ist ein weiterer Verfahrensschritt gezeigt, bei dem nach dem Arretieren über der Öffnung 51 der Setzbuchse 5, mittels des Saugdorns 40, das Septum 1 währenddessen dieses in der Ansaugkavität 41 angesaugt ist, durch den Setzkanal 52 der Setzbuchse 5 hindurchgeschoben wird und während des Hindurchschiebens auf Grund des sich verjüngenden Querschnitts des Setzkanals 52 das Septum 1 elastisch immer mehr verdichtet wird.

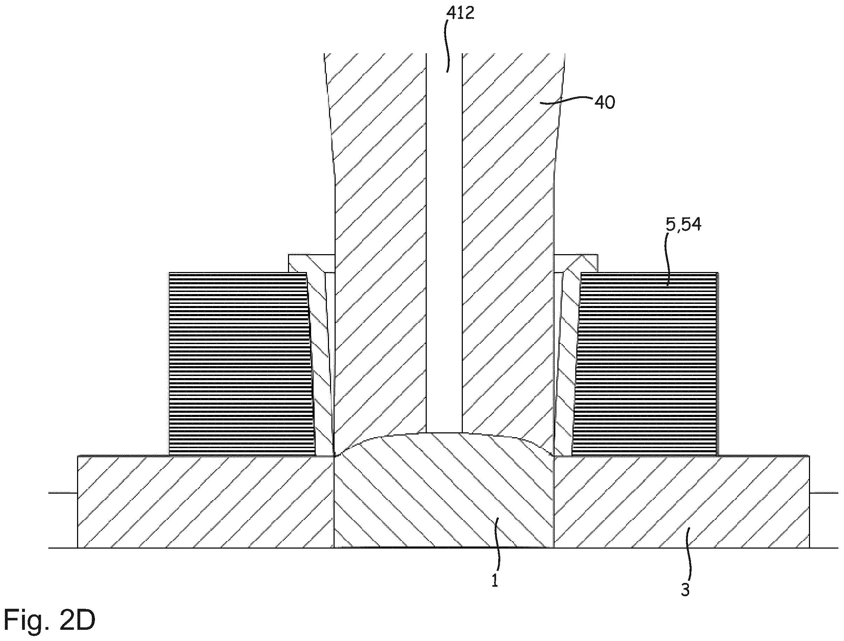

In der Figur 2D ist in einem weiteren Verfahrensschritt gezeigt, wie das nunmehr verdichtete Septum 1 mittels des Saugdorns 40 in die Setzöffnung 2, welche vorliegend eine Bohrung in dem Trägerelement 3 darstellt, eingeschoben und beispielsweise eingepresst wird und dort auch verbleibt, sodass vorliegend in besonders einfacher Art und Weise ein sowohl kostengünstiges als auch zeitsparendes, und insbesondere beispielsweise vollautomatisches, Setzverfahren für das Einfügen eines Septums 1 in eine Setzöffnung 2 eines Trägerelements 3 angeboten ist.

Nach dem Einschieben des Septums 1 in die Setzöffnung 2 des Trägerelements 3 wird zuerst die Setzbuchse 5 von dem Trägerelement 3 und erst anschließend der Saugdorn 40 von dem Septum 1 entfernt, was gewährleistet, dass das Septum 1 besonders sicher und ohne mechanische Schädigungen, beispielsweise bündig, in der Setzöffnung 2 verbleibt. Alternativ können auch der Saugdorn 40 und die Setzbuchse 5 gleichzeitig entfernt werden.

Vorzugsweise handelt es sich bei dem Septum 1 um einen in einer Draufsicht rund ausgebildetes, beispielsweise zwei-lagiges Septum 1, welches beispielsweise mit einem Gummi und/oder Gummigemisch gebildet ist. Ein derartiges Septum 1 ist besonders homogen und gleichmäßig zum einen elastisch verdichtbar und zum anderen kostengünstig herstellbar und handhabbar. Insbesondere ist in jedem Ausführungsbeispiel der Durchmesser der Setzöffnung 2 kleiner als der Durchmesser des Septums 1, damit das Septum 1 in der Setzöffnung 2 stets auch eingespannt bleibt. Denkbar ist insbesondere, dass eine Höhe des Septums 1 stets größer ist als eine Tiefe der Setzöffnung 2, sodass auch in vertikaler Richtung das Septum 1 in die Setzöffnung 2 eingepresst und elastisch verdichtet sein muss, um beispielsweise eine ebene Oberfläche ohne Vorsprünge ausbilden zu können.

Die Erfindung ist nicht durch die Beschreibung an Hand der Ausführungsbeispiele beschränkt. Vielmehr umfasst die Erfindung jedes neue Merkmal sowie jede Kombination von Merkmalen, was insbesondere jede Kombination von Merkmalen in die Patentansprüche beinhaltet, auch wenn dieses Merkmal oder diese Kombination selbst nicht explizit in den Patentansprüchen oder in den Ausführungsbeispielen angegeben ist.

### Bezugszeichenliste

- 1: Septum
- 2: Setzöffnung
- 3: Trägerelement
- 4: Transporttisch
- 5: Setzbuchse
- 6: Führung der Setzbuchse
- V: Richtung
- 40: Saugdorn
- 41: Ansaugkavität
- 51: Öffnung
- 52: Setzkanal
- 53: innere Setzbuchse
- 54: Mantel
- 55: Einsatzöffnung
- 100: Vorrichtung
- 411: Innenwand
- 412: Ansaugkanal
- 522: Innenfläche
- 4A: Werkzeugtisch
- 4B: Stempel
- 4C: Aktor und Aktorführung
- 4D: Haltevorrichtung
- 4E: Halterungen
- 40A: Öffnung

## Patentansprüche

1. Verfahren zum Einfügen zumindest eines Septums (1) in eine Setzöffnung (2) eines Trägerelements (3),
**dadurch gekennzeichnet, dass**
das Septum (1) in einem ersten Schritt mittels eines Saugdorns (40) an diesem durch Ansaugen befestigt wird;
in einem zweiten Schritt das Septum (1) über eine Öffnung (51) einer in vertikaler Richtung (V) zwischen dem Saugdorn (40) und dem Trägerelement (3) angeordneten Setzbuchse (5) angeordnet und nach einem bündigen Anordnen der Setzbuchse (5) auf dem Trägerelement (3) das Septum (1) durch einen sich ausgehend von der Öffnung (51) in Richtung des Trägerelements (3) in seinem Querschnitt verjüngenden Setzkanal (52) der Setzbuchse (5) mittels des Saugdorns (40) hindurch geschoben und in die Setzöffnung (2) des Trägerelements (3) eingeschoben wird, wobei während des Hindurchschiebens des Septums (1) durch den Setzkanal (52) das Septum (1) zumindest in lateraler Richtung elastisch verdichtet wird, um das Septum (1) in der Setzöffnung (2) einzuspannen.

2. Verfahren nach Anspruch 1,
**dadurch gekennzeichnet, dass**
nach dem Einschieben des Septums (1) in die Setzöffnung (2) des Trägerelements (3) zuerst die Setzbuchse (5) von dem Trägerelement (3) und erst anschließend der Saugdorn (40) von dem Septum (1) entfernt wird.

3. Verfahren nach Anspruch 1,
**dadurch gekennzeichnet, dass**
nach dem Einschieben des Septums (1) in die Setzöffnung (2) des Trägerelements (3) die Setzbuchse (5) von dem Trägerelement (3) und der Saugdorn (40) von dem Septum (1) gleichzeitig entfernt werden.

4. Verfahren nach zumindest einem der vorhergehenden Ansprüche,
**dadurch gekennzeichnet, dass**
zum Ansaugen und Arretieren des Septums (1) an dem Saugdorn (40) dieser eine Ansaugkavität (41) aufweist, wobei die Ansaugkavität (41) eine relativ zum Transporttisch (4) sich wegkrümmende Innenwand (411) aufweist, und wobei in der Innenwand (411) zumindest ein Ansaugkanal (412) mündet, mittels dem das Septum (1) an die Ansaugkavität (41) angesaugt und an dieser gehalten werden kann.

5. Verfahren nach zumindest einem der vorhergehenden Ansprüche,
**dadurch gekennzeichnet, dass**
die Öffnung (51) in der Setzbuchse (5) durch eine in der Setzbuchse (5) ausgebildete in Richtung des Transporttisches (4) sich im Querschnitt verjüngende Einsatzöffnung (55) gebildet ist.

6. Verfahren nach zumindest einem der vorhergehenden Ansprüche,
**dadurch gekennzeichnet, dass**
das Septum (1) eine runde oder ovale Querschnittsfläche aufweist, wobei eine Querschnittfläche des Setzkanals (51) ebenfalls rund oder oval ausgebildet ist.

7. Verfahren nach zumindest einem der vorhergehenden Ansprüche,
**dadurch gekennzeichnet, dass**
eine Innenfläche (522) des Setzkanals (52) der Setzbuchse (5) mit einem Teflon gebildet oder mit einem Teflon beschichtet ist.

8. Verfahren nach Anspruch 7,
**dadurch gekennzeichnet, dass**
die Setzbuchse (5) mit einer inneren Setzbuchse (53) und einem in horizontaler Richtung die innere Setzbuchse (53) zumindest teilweise ummantelnden äußeren Mantel (54) gebildet ist, wobei die Innenfläche (522) des Setzkanals (52) vollständig durch die innere Setzbuchse (53) gebildet ist.

9. Verfahren nach Anspruch 8,
**dadurch gekennzeichnet, dass**
der äußere Mantel (54) mit einem Metall, insbesondere Stahl, gebildet ist.

10. Verfahren nach zumindest einem der vorhergehenden Ansprüche,
**dadurch gekennzeichnet, dass**
das Trägerelement (3) mit zumindest einem Kunststoff gebildet ist.

11. Vorrichtung (100) zum Einfügen zumindest eines Septums (1) in eine Setzöffnung (2) eines Trägerelements (3),
**dadurch gekennzeichnet, dass**
das Septum (1) mittels eines Saugdorns (40) an diesem durch Ansaugen befestigbar ist;
das Septum (1) über eine Öffnung (51) einer in vertikaler Richtung (V) zwischen dem Saugdorn (40) und dem Trägerelement (3) angeordneten Setzbuchse (5) anordenbar und nach einem bündigen Anordnen der Setzbuchse (5) auf dem Trägerelement (3) das Septum (1) durch einem sich ausgehend von der Öffnung (51) in Richtung des Trägerelements (3) in seinem Querschnitt verjüngenden Setzkanal (52) der Setzbuchse (5) mittels des Saugdorns (40) hindurch schiebbar und in die Setzöffnung (2) des Trägerelements (2) einschiebbar ist, wobei der Setzkanal (52) derart ausgebildet ist, dass während des Hindurchschiebens des Septums (1) durch den Setzkanal (52) das Septum (1) zumindest in lateraler Richtung elastisch verdichtet wird, um das Septum (1) in der Setzöffnung (2) einzuspannen.

## Claims

1. Method for inserting at least one septum (1) into a placement opening (2) of a support element (3), **characterised in that**, in a first step, the septum (1) is fastened to a suction mandrel (40) by means of said suction mandrel by being suctioned thereon, in a second step, the septum (1) is arranged above an opening (51) of a placement sleeve (5) arranged between the suction mandrel (40) and the support element (3) in a vertical direction (V), and, after the placement sleeve (5) has been arranged so as to be flush on the support element (3), the septum (1) is pushed by means of the suction mandrel (40) through a placement channel (52) of the placement sleeve (5), the cross section of which placement channel tapers from the opening (51) towards the support element (3), and is pushed into the placement opening (2) of the support element (3), the septum (1) being resiliently compressed, at least in the lateral direction, while the septum (1) is being pushed through the placement channel (52), in order to clamp the septum (1) in the placement opening (2).

2. Method according to claim 1, **characterised in that**, after the septum (1) has been pushed into the placement opening (2) of the support element (3), first the placement sleeve (5) is removed from the support element (3) and only then is the suction mandrel (40) removed from the septum (1).

3. Method according to claim 1, **characterised in that**, after the septum (1) has been pushed into the placement opening (2) of the support element (3), the placement sleeve (5) is removed from the support element (3) and, at the same time, the suction mandrel (40) is removed from the septum (1).

4. Method according to at least one of the preceding claims, **characterised in that**, in order to suction the septum (1) onto the suction mandrel (40) and to lock it thereon, said suction mandrel comprises a suction cavity (41), the suction cavity (41) comprising an internal wall (411) that curves away relative to the transportation table (4), and at least one suction channel (412) opening in the internal wall (411), by means of which suction channel the septum (1) can be suctioned onto the suction cavity (41) and held thereon.

5. Method according to at least one of the preceding claims, **characterised in that** the opening (51) in the placement sleeve (5) is formed by an insertion opening (55) that is formed in the placement sleeve (5), the cross section of which insertion opening tapers towards the transportation table (4).

6. Method according to at least one of the preceding claims, **characterised in that** the septum (1) has a round or oval cross-sectional surface, a cross-sectional surface of the placement channel (51) also being round or oval.

7. Method according to at least one of the preceding claims, **characterised in that** an inner surface (522) of the placement channel (52) of the placement sleeve (5) is formed of a Teflon or is coated with a Teflon.

8. Method according to claim 7, **characterised in that** the placement sleeve (5) is formed having an inner placement sleeve (53) and an outer casing (54) that encloses the inner placement sleeve (53) at least in part in a horizontal direction, the inner surface (522) of the placement channel (52) being formed completely by the inner placement sleeve (53).

9. Method according to claim 8, **characterised in that** the outer casing (54) is formed of a metal, in particular steel.

10. Method according to at least one of the preceding claims, **characterised in that** the support element (3) is formed of at least one plastics material.

11. Device (100) for inserting at least one septum (1) into a placement opening (2) of a support element (3), **characterised in that** the septum (1) can be fastened to a suction mandrel (40) by means of said suction mandrel by being suctioned thereon, the septum (1) can be arranged above an opening (51) of a placement sleeve (5) arranged between the suction mandrel (40) and the support element (3) in a vertical direction (V), and, after the placement sleeve (5) has been arranged so as to be flush on the support element (3), the septum (1) can be pushed by means of the suction mandrel (40) through a placement channel (52) of the placement sleeve (5), the cross section of which placement channel tapers from the opening (51) towards the support element (3), and can be pushed into the placement opening (2) of the support element (2), the placement channel (52) being formed such that the septum (1) is resiliently compressed, at least in the lateral direction, while the septum (1) is being pushed through the placement channel (52), in order to clamp the septum (1) in the placement opening (2).

## Revendications

1. Procédé d'insertion sur au moins un septum (1) dans une ouverture de pose (2) d'un élément de support (3),
**caractérisé par le fait que**
le septum (1) est fixé par aspiration contre celui-ci au moyen d'un mandrin d'aspiration (40) dans une première étape ;
dans une deuxième étape le septum (1) est disposé au-dessus d'une ouverture (51) d'un manchon de pose (5) disposé en direction verticale (V) entre le mandrin d'aspiration (40) et l'élément de support (3) et après une disposition en affleurement du manchon de pose (5) sur l'élément de support (3), le septum (1) est introduit par poussée au moyen du mandrin d'aspiration (40) à travers un canal de pose (52) du manchon de pose (5) se rétrécissant dans sa section transversale en partant de l'ouverture (51) en direction de l'élément de support (3) et est introduit dans l'ouverture de pose (2) de l'élément de support (3), où, durant l'introduction par poussée du septum (1) à travers le canal de pose (52), le septum (1) est comprimé élastiquement au moins en direction latérale, pour serrer le septum (1) dans l'ouverture de pose (2).

2. Procédé selon la revendication 1,
**caractérisé par le fait qu'**
après l'introduction du septum (1) dans l'ouverture de pose (2) de l'élément de support (3) d'abord le manchon de pose (5) est retiré de l'élément de support (3) et ensuite seulement le mandrin d'aspiration (40) est retiré du septum (1).

3. Procédé selon la revendication 1,
**caractérisé par le fait qu'**
après l'introduction du septum (1) dans l'ouverture de pose (2) de l'élément de support (3), simultanément le manchon de pose (5) est retiré de l'élément de support (3) et le mandrin d'aspiration (40) est retiré du septum (1).

4. Procédé selon au moins l'une des revendications précédentes,
**caractérisé par le fait que**
pour l'aspiration et l'arrêt du septum (1) contre le mandrin d'aspiration (40), celui-ci présente une cavité d'aspiration (41), la cavité d'aspiration (41) présentant une paroi intérieure (411) se recourbant en s'éloignant par rapport au plateau de transport (4), et, dans la paroi intérieure (411), débouchant au moins un canal d'aspiration (412), au moyen duquel le septum (1) peut être aspiré contre la cavité d'aspiration (41) et maintenu contre celle-ci.

5. Procédé selon au moins l'une des revendications précédentes,
**caractérisé par le fait**
**que** l'ouverture (51) est formée dans le manchon de pose (5) à travers une ouverture d'introduction (55) se rétrécissant en section transversale en direction du plateau de transport (4), formée dans le manchon de pose (5).

6. Procédé selon au moins l'une des revendications précédentes,
**caractérisé par le fait que**
le septum (1) présente une surface de section transversale ronde ou ovale, une surface de section transversale du canal de pose (51) étant également formée ronde ou ovale.

7. Procédé selon au moins l'une des revendications précédentes,
**caractérisé par le fait qu'**
une surface intérieure (522) du canal de pose (52) du manchon de pose (5) est constituée par un Téflon ou est revêtue par un Téflon.

8. Procédé selon la revendication 7,
**caractérisé par le fait que**
le manchon de pose (5) est formé par un manchon de pose intérieur (53) et une enveloppe extérieure (54) enveloppant au moins partiellement le manchon de pose intérieur (53) en direction horizontale, la surface intérieure (522) du canal de pose (52) étant formée entièrement par le manchon de pose intérieur (53).

9. Procédé selon la revendication 8,
**caractérisé par le fait que**
l'enveloppe extérieure (54) est formée par un métal, en particulier de l'acier.

10. Procédé selon au moins l'une des revendications précédentes,
**caractérisé par le fait que**
l'élément de support (3) est formé par au moins une matière plastique.

11. Dispositif (100) pour l'insertion d'au moins un septum (1) dans une ouverture de pose (2) d'un élément de support (3),
**caractérisé par le fait que**
le septum (1) est apte à être fixé par aspiration contre celui-ci au moyen d'un mandrin d'aspiration (40) ;
le septum (1) est apte à être disposé au-dessus d'une ouverture (51) d'un manchon de pose (5) disposé en direction verticale (V) entre le mandrin d'aspiration (40) et l'élément de support (3) et après une disposition en affleurement du manchon de pose (5) sur l'élément de support (3), le septum (1) est apte à être introduit par poussée au moyen du mandrin d'aspiration (40) à travers un canal de pose (52) du manchon de pose (5) se rétrécissant dans sa section transversale en partant de l'ouverture (51) en direction de l'élément de support (3) et est apte à être introduit dans l'ouverture de pose (2) de l'élément de support (3), le canal de pose (52) étant réalisé de telle sorte que, durant l'introduction par poussée du septum (1) à travers le canal de pose (52), le septum (1) est comprimé élastiquement au moins en direction latérale, pour serrer le septum (1) dans l'ouverture de pose (2).
